# EUROPÄISCHE PATENTSCHRIFT

(11) **EP 2 622 967 B1**
(45) Veröffentlichungstag und Bekanntmachung des Hinweises auf die Patenterteilung: **13.05.2015**
(21) Anmeldenummer: 12153570.2
(22) Anmeldetag: 02.02.2012
(51) Int. Cl.: A23G 1/00, A23L 1/308, A23L 2/52, C12M 1/107, A23L 1/36, A23L 1/10, A23L 1/105, A23L 1/172, C12M 1/00

(54) **Verfahren zur Verarbeitung von pflanzlichen Rückständen**
Device for processing plant remnants
Procédé de traitement de résidus végétaux

(43) Veröffentlichungstag der Anmeldung: 07.08.2013
(73) Patentinhaber: Bühler Barth AG, 71691 Freiberg (DE)
(72) Erfinder: Lohmüller, Tobias, 71688 Freiberg (DE)
(74) Vertreter: Hepp Wenger Ryffel AG

(56) Entgegenhaltungen:
- EP-A1- 1 886 578
- EP-A1- 2 022 335
- US-A- 5 622 738

## Beschreibung

Die Erfindung betrifft ein Verfahren und eine Anlage zur Verarbeitung von pflanzlichen Rückständen, insbesondere von Schalen von Samen und Nüssen, weiter insbesondere von Schalen von Kakaobohnen, Schalen von Getreidesaat und Reisrückständen.

Aus dem Stand der Technik sind viele Vorschläge bekannt, Nahrungs- und Genussmitteln unverdauliche Ballaststoffe wie Kleie, Carboxylmethylzellulose, pektingeschichtete Zellulose, Lignin, Hemizellulosen, Pentosanen, Gummi und Pektine zuzusetzen.

Auch Nahrungsfasern aus Kakaoschalen und anderen Pflanzenbestandteilen wie Getreideschalen, Rückstände von Reiskörnern, Keimen und/oder Nüssen wurden, z. B. wegen ihres Zellulosegehalts, als möglicher Nahrungsbestandteil erkannt.

Als Kakaoschale wird die äussere Umhüllung der Kakaobohnen bezeichnet, auch Testa genannt. Nach der Ernte werden die Kakaofrüchte im Schnitt sechs Tage fermentiert, wobei sich das Fruchtfleisch von den Bohnen löst und die Bohnen ihren gewünschten Geschmack und die braune Färbung entwickeln. Anschliessend werden die Bohnen getrocknet, gereinigt und geröstet. Die Schalen der Bohnen werden gebrochen und von den Bohnen getrennt.

Die Kakaoschalen werden in der Regel als Abfallprodukt betrachtet. Die Kakaoschalen enthalten allerdings auch wertvolle Inhaltsstoffe wie z. B. Polyphenole (1 bis 2 %), Alkaloide wie Theobromin (1 bis 2 %), Vitamine wie Vitamin D, Minerale, Aminosäuren und lösliche wie unlösliche diätische Fasern. Die Kakaoschalen enthalten immer noch bis zu 6 % Fett.

Die Verwendung von Kakaoschalen in Nahrungsmitteln ist vielerorts gesetzlich beschränkt. Zwar ist seit der Umsetzung der EU-Richtlinie 2000/36/EG (2000) z. B. in Deutschland keine Höchstmenge mehr definiert, dennoch sind Hersteller und Verbraucher noch immer an einer Überwachung des Schalenanteils interessiert, da diese gesundheitsgefährdende Stoffe wie Pestizide, Mikroorganismen, Mykotoxine, freie Fettsäuren (FFA) und Schwermetalle enthalten können, und die Schalen Schäden an den Walzenstühlen verursachen können.

Insbesondere Kakaoschalenfasern haben ausserdem zumeist schwellende bzw. gelifizierende Eigenschaften. Sie können Fett sowie Wasser binden und somit die Viskosität der Mischung beeinflussen, der sie zugesetzt sind. Die Kakaoschalenfasern sind daher bislang nur für die Verwendung in wenigen bestimmten Nahrungsmitteln geeignet. Die Verwendung von Kakaoschalenfasern in einem gesäuerten Lebensmittelprodukt wie z. B. einem Käse ist beispielsweise in EP 2 174 555 gezeigt.

Es ist zudem bekannt, Kakaoschalen in aromatischen Getränken, Kakaoprodukten, Mulch, Dünger und Tiernahrung zu verwenden.

Die Gewinnung von Nahrungsfasern aus Kakaoschalen ist beispielsweise in EP 0 068 229 gezeigt, wobei die Schalen der Kakaobohnen einer Nassreinigung unterworfen, getrocknet und gemahlen werden. Das fein gemahlene Endprodukt soll Nahrungs- und Genussmitteln zur Verbesserung der verdauungsfördernden Eigenschaften zugesetzt werden.

Die mechanische Zerkleinerung von Kakaobohnenschalen ist aus EP 1 733 624 bekannt. Die Druckschrift zeigt ein Verfahren zum Mahlen von Kakaoschalen, wobei die Kakaoschalen in einer Wirbelverarbeitungsvorrichtung, auch "jet-mill" genannt, in der eingeführten Luft mitgerissen und vermahlen werden. Andere Verfahren wie die Zermahlung in einer Windsichtermühle sind ebenfalls möglich, führen aber zu einem höheren Verschleiss.

Die Verwendung von Kakaoschalenextrakten ist ebenfalls bekannt. Ein Lebensmittelfarbstoff kann beispielsweise gemäss US 4,156,030 durch die Extraktion mit einer sauren Ethanollösung gewonnen werden.

Pigmente können gemäss US 4,532,147 auch mit Hilfe eines wässrigen Alkohols extrahiert werden.

Ein Schokoladengeschmacksstoff kann durch die Behandlung von Kakaoschalen mit einem alkalisierenden Agens zur Verfügung gestellt werden, wie in EP 2 174 557 gezeigt wird.

Die bekannten Behandlungsmethoden von Schokoladenschalen zum Bereitstellen von Nahrungsmittelzusätzen modifizieren vor allem das Umfeld der Kakaoschalenfasern, lassen die Zusammensetzung aber im Wesentlichen unverändert. Ein gezieltes Abtrennen von unerwünschten Bestandteilen findet nicht statt.

Des Weiteren ist aus EP 0 328 019 ein Verfahren bekannt, mit welchem aus hochwertigen Ausgangsstoffen diätische Kakaonahrungsfasern für die Herstellung von nahrungsfaserreichen Schokoladenprodukten bereitgestellt werden.

Dazu wird ein Kakaopulver mit geringem Fettgehalt oder ein Presskuchen (Kakaoliquor, dem die Kakaobutter entzogen ist) enzymatisch behandelt, die beim Abbau der Stärke anfallenden Stärkeabbauprodukte separiert und der feste Rückstand gewaschen und getrocknet.

Bei dem herkömmlichen Herstellungsprozess von Schokolade fallen ca. 15-20 % (Gewicht) Abfall an Kakaoschalen an.

Ähnlich verhält es sich mit den Rückständen anderer pflanzlicher Nahrungsmittel wie den Schalen von anderen Samen und Nüssen, etwa von Reis, Getreide, Bohnen und Keimen. Diese fallen ebenfalls bei dem herkömmlichen Produktionsprozess in beträchtlicher Menge an. Sie enthalten in der Regel Fette, die oxidieren und sind somit nicht für den Verzehr geeignet.

Es stellt sich daher die Aufgabe, eine Methode zur Weiterverarbeitung der pflanzlichen Rückstände vorzustellen, mit welcher die Nachteile des bekannten überwunden werden und ein wertiges Endprodukt zur Verfügung gestellt wird.

Die Aufgabe wird gelöst durch ein Verfahren gemäss Anspruch 1. Das Verfahren dient zur Verarbeitung von pflanzlichen Rückständen, insbesondere Schalen von Samen und Nüssen, weiter insbesondere von Schalen von Kakaobohnen, Schalen von Getreidesaat und Reisrückständen, Rückständen von ölhaltigen Keimen und Nüssen. Insbesondere werden mit dem Verfahren diätischen Fasern und/oder Ausgangsstoffe für eine Biogasanlage erzeugt.

Unter diätischen Fasern werden hier unverdauliche, für Nahrungsmittel geeignete Ballaststoffe verstanden.

Das erfindungsgemässe Verfahren umfasst die Verfahrensschritte (i) Bereitstellen von pflanzlichen Rückständen mit einem Schalenanteil von mindestens 20 Gew.-%, insbesondere mindestens 50 Gew.-%, weiter insbesondere mindestens Gew.-90 %, (ii) zumindest teilweise Hydrolyse von Bestandteilen der pflanzlichen Rückstände, insbesondere zumindest teilweise Hydrolyse von einem Kohlenhydrat, einem Fett und/oder einem Protein und (iii) insbesondere Trennung von flüssiger Phase mit gelösten Bestandteilen und fester Phase.

Unter Hydrolyse wird im vorliegenden Verfahren insbesondere ein Prozess verstanden, bei welchen mindestens 5 Gew.-%, bevorzugt mindestens 10 Gew.-% der Proteine, Fette und/oder Kohlenhydrate der pflanzlichen Rückstände abgebaut werden.

Ausgangsmaterial für das Verfahren sind pflanzliche Rückstände, die in der Regel als Abfall bei einem herkömmlichen Herstellungsprozess anfallen, wie zum Beispiel vor oder nach dem Rösten von den Kakaobohnen abgetrennte Samenschalen, auch Kakaoschalen genannt, Rückstände von Reiskörnern, Maiskörnern, Kaffeebohnen, Weizen- oder anderen Getreidekörnern.

Da sich im herkömmlichen Herstellungsprozess die wertigen Bestandteile von den Rückständen nicht komplett trennen lassen, enthält auch der Abfallanteil stets einen gewissen Prozentsatz des wertigen Anteils, also des Kerns, des Fruchtfleisches, des Keims oder der Bohne. Dieser Anteil kann zum Beispiel bei Beginn oder am Ende einer Herstellungscharge auch grösser sein. Erfindungsgemäss enthalten die pflanzlichen Rückstände einen Schalenanteil von mindestens 20 Gew.-%, insbesondere mindestens 50 Gew.-%, weiter insbesondere mindestens 90 Gew.-%.

Bei der Hydrolyse erfolgt eine Aufspaltung von chemischen Bestandteilen durch die Anlagerung eines Wassermoleküls.

In einer wässrigen Lösung gehen zum einen die ohnehin löslichen Bestanteile der pflanzlichen Rückstände in Lösung, zudem aber auch jene Bestandteile, die erst durch die Hydrolyse zu löslichen Bestandteile werden.

Die in Lösung gegangenen Bestandteile werden bevorzugt bei der anschliessenden Trennung von flüssiger und fester Phase von dem Rückstand separiert.

Die Trennung erfolgt bevorzugt durch Filtration, durch Zentrifugieren, durch Dekantieren und/oder durch Trocknung.

Vorzugsweise wird die Suspension mit einem Schneckenförderer, einem Extruder oder Expeller über ein Lochblech geführt, wobei ein Grossteil der Flüssigkeit abläuft. Anschliessend wird die Masse unter Druck verdichtet, wobei ein Restfeuchtegehalt von etwa 12 Gew.-% erreicht wird.

Nachfolgend kann die Masse unter Erwärmung und/oder Trockenluftzufuhr getrocknet werden, sodass ein Restfeuchtegehalt von 3-4 Gew.-% übrig bleibt. Die resultierende Masse kann weiter verarbeitet werden, sie ist beispielsweise leicht vermahlbar.

Die feste Phase weist signifikant geringere Anteile von wasserlöslichen Salzen auf, weniger Proteine, weniger Fette und weniger Zellulose als die pflanzlichen Rückstände, die Ausgangsstoff für die Hydrolyse waren. Die feste Phase besteht im Wesentlichen aus wasserunlöslichen, also weitgehend unverdaulichen Fasern. Die relativ geschmacksneutrale, im Falle von Kakaoschalen braune feste Phase kann als Nahrungsfaser eingesetzt werden. Die feste Phase ist praktisch nicht mehr gelifizierend.

Durch Zugabe von Nährstoffen lässt sich mit der Hydrolyse und dem nachfolgenden Abtrennen der wasserlöslichen Bestandteile ein Verhältnis der Makronährstoffe (C:N:P:S, d. h. das Mengenverhältnis von Kohlenstoff, Stickstoff, Phosphor und Schwefel) von 500:15:5:3 herstellen, das C:N Verhältnis liegt bevorzugt zwischen 10 und 45.

Bevorzugt wird für die Hydrolyse eine Suspension der pflanzlichen Rückstände in einem Lösungsmittel, insbesondere Wasser hergestellt, insbesondere mit einem Anteil von bis zu 40 Gew.-% Trockenmasse.

Die Hydrolyse erfolgt bevorzugt in einem abschliessbaren, temperierbaren und druckbeaufschlagbaren Tank. Typischerweise kann 100-300 m³ Suspension in einem Tank verarbeitet werden.

Die Suspension wird bevorzugt bei mittlerer Rührgeschwindigkeit vermischt, sodass eine möglichst homogene Vermischung bewirkt wird. Das Rühren sorgt für einen ausreichenden Stoffaustausch und verhindert eine Sedimentation. Je länger dem Hydrolyseprozess Zeit gegeben wird, desto geringer kann die Rührgeschwindigkeit sein. Je schneller der Prozess erfolgen soll, umso höher muss die Rührgeschwindigkeit sein.

Die Hydrolyse wird durch geeignete Temperatur- und Druckbedingungen begünstigt. Vorteilhafterweise findet die Hydrolyse in einem Tank bei einer Temperatur zwischen 25 und 40 °C, insbesondere zwischen 30 und 38 °C, und bei Umgebungsdruck statt.

Die Hydrolyse erfordert typischerweise eine Dauer von bis zu 7 Tagen, bevorzugt von bis zu 5 Tagen, weiter bevorzugt von bis zu 1-2 Tagen, insbesondere von mindestens 3 Stunden.

Die Hydrolyse wird des Weiteren begünstigt durch den Zusatz von Enzymen, insbesondere von Hydrolasen wie Lipasen, Amylasen und Proteasen. Enzyme sorgen insbesondere für die katalytische Hydrolyse von Biomolekülen, also Sacchariden, Proteinen und Fetten, die in ihre Bausteine zerlegt werden.

Bevorzugt findet die Hydrolyse daher enzymatisch statt. Die meisten Hydrolysen laufen effektiver und schneller, wenn die Reaktion in einem sauren oder basischen Medium erfolgt.

In einer vorteilhaften Ausführungsform des erfindungsgemässen Verfahrens erfolgt die Hydrolyse unter dem Zusatz einer Säure, insbesondere einer organischen Säure wie Essigsäure oder Ameisensäure oder einer Base, insbesondere eines Phosphatpuffers, eines Carbonatpuffers, NaOH oder KOH.

Dabei wird insbesondere ein pH-Wert zwischen 3.0 und 6.5, bevorzugt zwischen 3.5 und 5.5 eingestellt.

Typischerweise fällt der pH-Wert während der Hydrolyse von einem Wert von ca. 7 auf etwa 3.5.

Die Hydrolyse kann auch unter Einwirkung von Mirkoorganismen ablaufen. Dazu kann einerseits die vorhandene Flora des zu hydrolysierenden Materials verwendet werden oder es werden gezielt zur Hydrolyse fähige Mikroorganismen zugesetzt. Bevorzugt werden für die Hydrolyse in dem erfindungsgemässen Verfahren Mikroorganismen, insbesondere ein Impfbakterium für Kompost, zugesetzt, insbesondere in der Menge 1/10000, bevorzugt 1/1000 (Bakteriumslösung zu Suspension).

Um die feste Phase für den Einsatz in der Herstellung von Nahrungsmitteln vorzubereiten, wird in einem nachfolgenden Schritt vorteilhafterweise der Feststoffanteil gewaschen, sterilisiert und/oder getrocknet.

Die Debakterisierung kann in einem Röster erfolgen, in welchem die thermische Behandlung gleichzeitig für eine Sterilisation und für eine Trocknung sorgt.

Bevorzugt wird der Feststoffanteil zusätzlich gemahlen. Dies kann vor dem Waschen, Trocknen und Debakterisieren, vor dem Trocknen oder sogar vor dem Waschen geschehen.

Der Feststoffanteil kann ausserdem zusätzlich gefärbt werden, beispielsweise durch Alkalisierung.

Feste Phase, die aus der Hydrolyse von Kakaoschalen gewonnen wird, also nahrungsfaserreiches Kakaomaterial, ist geeignet zur Ergänzung bei der Herstellung von Schokoladen, Compounds und/oder Fillings (z. B. dunkler Schokolade und besonders für geformte Schokolade und Kuvertüre), Kakaogetränken, Konfektriegeln, Schokoladenaufstrich und Backwaren.

Es ist bekannt, dass Zellulose bei einem pH-Wert von 7.5, der für die Methanbildung optimal ist, kaum abgebaut werden kann. Daher wird faserhaltige Biomasse in der Regel zunächst hydrolysiert und anschliessend fermentiert.

Der flüssigen Phase, die bei dem erfindungsgemässen Verfahren entsteht, ist bereits ein Grossteil der nicht abbaubaren Zellulose entzogen. Die flüssige Phase enthält vorwiegend Inhaltsstoffe in einer für die Methan bildenden Bakterien umsetzbaren Form. Sie kann daher direkt zur Biogaserzeugung eingesetzt werden.

Vorteilhafterweise umfasst das erfindungsgemässe Verfahren daher als weiteren Verfahrensschritt, dass die flüssige Phase als energiereiche Flüssigkeit in eine Biogasanlage überführt wird. Die Aufgabe wird ausserdem gelöst durch eine Verwendung des festen Anteils aus dem Verfahrensprodukt des Verfahrens wie oben beschrieben als diätische Faser.

Die Aufgabe wird ausserdem gelöst durch eine Verwendung des flüssigen Anteils aus dem Verfahrensprodukt des Verfahrens wie oben beschrieben als Beschickung für eine Biogasanlage, insbesondere für die Stromerzeugung.

Bei der Biogasentwicklung nutzen Mikroorganismen unter anaeroben Bedingungen (unter Luftausschluss) die in Kohlenhydraten, Fetten und Proteinen gespeicherte chemische Energie für ihren Stoffwechsel. Die Biogasgewinnung untergliedert sich in vier Stufen:
In der Hydrolysephase spalten fermentative Bakterien polymere Verbindungen wie Proteine, Fette und Kohlenhydrate mithilfe von Enzymen in einfachere Bestandteile (Monomere) wie z. B. Aminosäuren, Glukose und Fettsäure. Hieran sind in der Regel anaerobe Bakterien beteiligt.

In der säurebildenden Phase erfolgt eine Vergärung und Säurebildung. Die gelösten Stoffe werden durch fermentative Bakterien zu organischen Säuren (Essigsäure, Propionsäure, Buttersäure), niederen Alkoholen, Aldehyden, Wasserstoff, Kohlendioxid und anderen Gasen wie Ammoniak und Schwefelwasserstoff abgebaut. Dieser Vorgang erfolgt, bis die Bakterien durch ihre eigenen Abbauprodukte in ihrem Abbauprozess gehemmt werden (niedriger pH-Wert).

Die dritte, so genannte acetogene Phase bildet das Bindeglied zwischen der Vergärung (Versäuerung) und der Methanbildung. Hier werden die Bestandteile durch acetogene Bakterien so aufbereitet, dass methanogene Bakterien diese in Methan umwandeln können. Die Reaktion verläuft endotherm, es muss also Wärme zugeführt werden.

In der vierten Phase, der Methanogenese wird die Essigsäure durch extrem sauerstoffempfindliche methanogene Bakterien zu Methan, Kohlendioxid und Wasser gespalten. Das Wasser wird während der Kondensation dem Biogasgemisch entnommen.

30 % der bekannten methanogenen Bakterienarten nutzen Wasserstoff und Kohlendioxid für ihren Stoffwechsel und sorgen so durch die Umsetzung des Wasserstoffs mit dem zuvor gebildeten Kohlendioxid für einen niedrigen Wasserstoffpartialdruck. Dieser ist für die Existenz der Essigsäurebakterien unerlässlich,obwohl sie selber Wasserstoff produzieren. Die methanogenen Bakterien und die acetogenen Bakterien leben somit in Symbiose.

Das entstandene Gas wird nach einer Zwischenpufferung in einem Gasspeicher in aller Regel in einem Blockheizkraftwerk zur Produktion von Strom und/oder Wärme verwendet. Alternativ kann das entstandene Gas direkt zur Verbrennung in einem thermischen Prozess genutzt werden, beispielsweise bei der Beheizung eines Rösters.

Der zu erwartende Gasertrag in der Biogasanlage beträgt pro Tonne Kakaoschalen zwischen 460 und 500 Normkubikmeter (460-500 Nm³/t) mit einer Methankonzentration bis zu 60% ± 5 %.

Die der Erfindung zugrunde liegende Aufgabe wird ausserdem gelöst durch ein Verfahren, insbesondere wie oben beschrieben, zur Verarbeitung von pflanzlichen Rückständen, insbesondere Schalen von Samen und Nüssen, weiter insbesondere Schalen von Kakaobohnen, Schalen von Getreidesaat und Reisrückständen, Rückständen von ölhaltigen Keimen und Nüssen, wobei aus zumindest einem Teil der pflanzlichen Rückstände in einer Biogasanlage Biogas erzeugt wird und zumindest ein Teil des Biogases zur Bereitstellung von Energie bei der Herstellung und/oder der Verarbeitung eines Produktes verwendet wird, bei welcher pflanzliche Rückstände übrig bleiben, die ihrerseits in dem Verfahren verarbeitet werden.

Bevorzugt wird das Biogas direkt in einem thermischen Verbrennungsprozess genutzt, bevorzugt in einem Röster.

In einer bevorzugten Ausführung des Verfahrens wird bei der Herstellung und/oder der Verarbeitung des Produktes, z. B. beim Rösten von Kakaobohnen bzw. beim Herstellen von Kakaonibs, ausser der durch das Biogas der pflanzlichen Rückstände bereitgestellten Energie keine weitere Energie zugeführt.

Das Verfahren ist somit ein autonomes Verfahren zum Verarbeiten eines pflanzlichen Produkts, insbesondere von Kakaobohnen.

Das erfindungsgemässe Verfahren findet in einer Anlage statt, die
- eine Produktionsvorrichtung, insbesondere einen Röster oder einen Röster und einen Hydrolysetank,
- einen Biogastank,
- eine Zufuhreinrichtung, über welche zumindest ein Teil von pflanzlichen Rückständen aus der Produktionsvorrichtung in den Biogastank überführbar ist, und
- eine Biogasverwertungseinrichtung, bei welcher aus dem im Biogastank gewonnen Biogas Energie zum Betreiben der Produktionseinrichtung gewinnbar ist,
umfasst.

Die Produktionsvorrichtung und der Biogastank sind bevorzugt räumlich nah beieinander, sodass als Zuführeinrichtung eine Rohrleitung oder eine Band verwendet werden kann.

Bei der Biogasverwertungseinrichtung kann es sich beispielsweise um eine Stromerzeugungsvorrichtung handeln oder um einen Brenner, je nachdem, in welcher Form die Energie bei der Produktion benötigt wird. Die Biogasverwertungseinrichtung kann in die Produktionsvorrichtung integriert sein, zum Beispiel in Form eines Brenners als Teil eines Rösters.

Die pflanzlichen Rückstände, die beispielsweise in dem Röster anfallen, können zunächst, insbesondere wie weiter oben beschrieben, in einem Hydrolysetank einer Hydrolyse unterzogen werden. Anschliessend wird aus der flüssigen Phase in dem Biogastank Biogas gewonnen.

### Ausführungsbeispiel:

150 kg Kakaoschalen mit einem Gehalt von etwa 1.5 ± 1 Gew.-% Fett, 4.5 ± 2.5 Gew.-% Wasser, 11 ± 6 Gew.-% Proteinen, 23 ± 7 Gew.-% Cellulose und Pentosane sowie 8 ± 3 Gew.-% Asche werden in 500 1 Regenwasser in einem Hydrolysetank mit 3 m³ Volumen suspendiert. Je nachdem, wie die Kakaoschalen gewonnen wurden und wie viel Fruchtanteil noch enthalten ist, kann der Fettgehalt auch wesentlich höher sein, z.B. bis zu 7 Gew.-%.

Die Suspension wird bei einer Temperatur von etwa 25 °C gehalten. Die Mischung wird alle vier Stunden eine Minute lang kräftig gerührt.

Nach einer Inkubationszeit von 24 Stunden werden Feststoffe und Flüssigkeit mittels eines Lochblechs unter Ausschluss von Sauerstoff getrennt, wobei der flüssige Anteil zunächst in einen Puffertank, der ebenfalls ein Volumen von 3 m³ aufweist, geleitet wird.

Die feste Fraktion wird in der Sonne bis zu einem Restfeuchtegehalt von 12 Gew.-% getrocknet. Danach wird der Feststoff pasteurisiert und thermisch getrocknet, bis er eine Restfeuchte von kleiner als 5 Gew.-% hat.

Danach erfolgt eine Vermahlung auf eine Pulverkorngrösse, bei welcher 99.5 % der Körner einen Durchmesser von kleiner als 75 µm haben.

Das feste Endprodukt ist ein nahrungsfaserreiches KakaoMaterial. Dieses nahrungsfaserreiche Kakaomaterial ist besonders geeignet zur Ergänzung bei der Herstellung von Schokoladencompounds und so genannten Fillings (z. B. Pralinen-, Konfekt- und/oder Brotfüllungen), Kakaogetränken, Konfektriegeln, Schokoladenaufstrich und Backwaren.

Durch die Behandlung ist der Fettanteil um 70 % reduziert worden. Das Verhältnis von wasserunlöslichen zu wasserlöslichen Nahrungsfasern beträgt für den Ausgangsstoff etwa 5.5 und ist für das Endprodukt grösser als 19.

Die flüssige Phase wird in einen dritten Biogastank geleitet, der 40 m³ Volumen fasst und in dem sich 30 m³ Flüssigkeit befinden. Überschüssige Flüssigkeit wird dann wieder in den ersten Tank geleitet, in welchen wiederum frische Kakaoschalen zugefügt werden und die Hydrolyse beginnt. Auf diese Weise entsteht ein geschlossener Flüssigkeitskreiskauf, was insbesondere für Anlagen in Ländern mit wenig Regenwasser vorteilhaft ist. Alternativ kann für die Hydrolyse stets frisches Regenwasser verwendet werden.

Die Temperatur im Biogastank beträgt ca. 38-40 °C. Es entsteht einen Biogasdruck von 2-5 bar.

Der Biogasertrag ergibt sich pro Tonne Ausgangsmasse Kakaoschale (o DM, "organic dry matter") zu 485 Normkubikmeter (485 Nm³/t o DM ± 10%) mit einer Methankonzentration von 60 % (± 5 %).

Der Biogasertrag reicht aus, um einen Röster für Kakaobohnen zu betreiben, in dem die Ausgangsmenge an Kakaoschalen anfällt.

## Patentansprüche

1. Verfahren zur Verarbeitung von pflanzlichen Rückständen, insbesondere Schalen von Samen und Nüssen, weiter insbesondere Schalen von Kakaobohnen, Schalen von Getreidesaat und Reisrückständen, Rückständen von ölhaltigen Keimen und Nüssen, insbesondere zur Erzeugung von diätischen Fasern und/oder von Ausgangsstoffen für eine Biogasanlage, **gekennzeichnet durch** die folgenden Verfahrensschritte
(i) Bereitstellen von pflanzlichen Rückständen mit einem Schalenanteil von mindestens 20 Gew.-%, insbesondere mindestens 30 Gew.-%, weiter insbesondere mindestens 50 Gew.-%, weiter insbesondere 90 Gew.-%,
(ii) zumindest teilweise Hydrolyse von Bestandteilen der pflanzlichen Rückstände, insbesondere zumindest teilweise Hydrolyse von einem Kohlenhydrat, einem Fett und/oder einem Protein,
(iii) Trennung von flüssiger Phase mit gelösten Bestandteilen und fester Phase,
(iv) Bereitstellung der festen Phase für den Einsatz in der Herstellung von Nahrungsmitteln, vorteilhafterweise **durch** Waschen, Sterilisieren und/oder Trocknen, und insbesondere Debakterisierung.

2. Verfahren gemäss Anspruch 1, **dadurch gekennzeichnet, dass** für die Hydrolyse eine Suspension der pflanzlichen Rückstände in einem Lösungsmittel, insbesondere Wasser, hergestellt wird, insbesondere mit einem Anteil von bis zu 40 Gew.-% Trockenmasse.

3. Verfahren gemäss Anspruch 1 oder 2, **dadurch gekennzeichnet, dass**
die Hydrolyse in einem Tank bei einer Temperatur zwischen 25 °C und 40 °C, insbesondere zwischen 30 °C und 38 °C stattfindet.

4. Verfahren gemäss einem der vorangehenden Ansprüche, **dadurch gekennzeichnet, dass**
die Hydrolyse enzymatisch, insbesondere unter Zusatz von Enzymen, stattfindet.

5. Verfahren gemäss einem der vorangehenden Ansprüche, **dadurch gekennzeichnet, dass**
die Hydrolyse bei einem pH-Wert zwischen 3.0 und 6.5, bevorzugt zwischen 3.5 und 5.5 stattfindet, insbesondere dass die Hydrolyse unter dem Zusatz einer Säure oder einer Base erfolgt.

6. Verfahren gemäss einem der vorangehenden Ansprüche, **dadurch gekennzeichnet, dass**
für die Hydrolyse Mikroorganismen, insbesondere ein Inokulum, zugesetzt werden.

7. Verfahren gemäss einem der vorangehenden Ansprüche 1-6,
**dadurch gekennzeichnet, dass** die flüssige Phase als energiereiche Flüssigkeit in eine Biogasanlage überführt wird.

8. Verwendung des festen Anteils aus dem Verfahrensprodukt des Verfahrens gemäss einem der Ansprüche 1-7 als diätische Faser.

9. Verwendung des flüssigen Anteils aus dem Verfahrensprodukt des Verfahrens gemäss zur Verarbeitung von pflanzlichen Rückständen, insbesondere Schalen von Samen und Nüssen, weiter insbesondere Schalen von Kakaobohnen, Schalen von Getreidesaat und Reisrückständen, Rückständen von ölhaltigen Keimen und Nüssen, insbesondere zur Erzeugung von diätischen Fasern und/oder von Ausgangsstoffen für eine Biogasanlage, mit den folgenden Verfahrensschritten als Beschickung für eine Biogasanlage, insbesondere für die Stromerzeugung:
(i) Bereitstellen von pflanzlichen Rückständen mit einem Schalenanteil von mindestens 20 Gew.-%, insbesondere mindestens 30 Gew.-%, weiter insbesondere mindestens 50 Gew.-%, weiter insbesondere 90 Gew.-%,
(ii) zumindest teilweise Hydrolyse von Bestandteilen der pflanzlichen Rückstände, insbesondere zumindest teilweise Hydrolyse von einem Kohlenhydrat, einem Fett und/oder einem Protein.

10. Verfahren zur Verarbeitung von pflanzlichen Rückständen, insbesondere Schalen von Samen und Nüssen, weiter insbesondere Schalen von Kakaobohnen, Schalen von Getreidesaat und Reisrückständen, Rückständen von ölhaltigen Keimen und Nüssen, insbesondere gemäss den Ansprüchen 1-7, mit den folgenden Schritten
(i) Erzeugung von Biogas in einer Biogasanlage aus zumindest einem Teil der pflanzlichen Rückstände,
(ii) Verwendung von zumindest einem Teil des Biogases zur Bereitstellung von Energie bei der Herstellung und/oder der Verarbeitung eines Produktes, bei welcher pflanzliche Rückstände übrig bleiben, die ihrerseits zumindest teilweise in dem Verfahren verarbeitet werden.

11. Anlage zur Verarbeitung von pflanzlichen Rückständen, insbesondere Schalen von Samen und Nüssen, weiter insbesondere Schalen von Kakaobohnen, Schalen von Getreidesaat und Reisrückständen, Rückständen von ölhaltigen Keimen und Nüssen, insbesondere gemäss Anspruch 10, umfassend
- eine Produktionsvorrichtung, insbesondere einen Röster oder einen Röster und einen Hydrolysetank,
- einen Biogastank,
- eine Zufuhreinrichtung, über welche zumindest ein Teil von pflanzlichen Rückständen aus der Produktionsvorrichtung in den Biogastank überführbar ist uhd
- eine Biogasverwertungseinrichtung, bei welcher aus dem im Biogastank gewonnen Biogas Energie zum Betreiben der Produktionseinrichtung gewinnbar ist.

## Claims

1. Method for processing plant remains, in particular shells of seeds and nuts, even more in particular shells of cocoa beans, shells of grain seed and rice remains, residues of oil-containing germs and nuts, in particular for producing dietary fibers and/or starting materials for a biogas plant, **characterized by** the following method steps:
(i) provision of plant remains with a shell fraction of at least 20% by weight, in particular at least 30% by weight, even more in particular at least 50% by weight, even more in particular 90% by weight,
(ii) at least partial hydrolysis of constituents of the plant remains, in particular at least partial hydrolysis of a carbohydrate, a fat and/or a protein,
(iii) separation of liquid phase with dissolved constituents and solid phase,
(iv) provision of the solid phase for use in producing foods, advantageously by washing, sterilizing and/or drying, and in particular debacterization.

2. Method according to Claim 1, **characterized in that** for the hydrolysis a suspension of the plant remains in a solvent, in particular water, is prepared, in particular with a fraction of up to 40% by weight dry mass.

3. Method according to Claim 1 or 2, **characterized in that**
the hydrolysis takes place in a tank at a temperature between 25°C and 40°C, in particular between 30°C and 38°C.

4. Method according to one of the preceding claims, **characterized in that**
the hydrolysis takes place enzymatically, in particular with the addition of enzymes.

5. Method according to one of the preceding claims, **characterized in that**
the hydrolysis takes place at a pH between 3.0 and 6.5, preferably between 3.5 and 5.5, in particular in that the hydrolysis takes place with the addition of an acid or of a base.

6. Method according to one of the preceding claims, **characterized in that**
microorganisms, in particular an inoculum, are added for the hydrolysis.

7. Method according to one of the preceding Claims 1-6, **characterized in that** the liquid phase is transferred as energy-rich liquid to a biogas plant.

8. Use of the solid fraction from the process product of the method according to one of Claims 1-7 as dietary fiber.

9. Use of the liquid fraction from the process product of the method for processing plant remains, in particular shells of seeds and nuts, even more in particular shells of cocoa beans, shells of grain seed and rice remains, residues of oil-containing germs and nuts, in particular for producing dietary fibers and/or starting materials for a biogas plant, having the following method steps as feed for a biogas plant, in particular for generating electricity:
(i) provision of plant remains with a shell fraction of at least 20% by weight, in particular at least 30% by weight, even more in particular at least 50% by weight, even more in particular 90% by weight,
(ii) at least partial hydrolysis of constituents of the plant remains, in particular at least partial hydrolysis of a carbohydrate, a fat and/or a protein.

10. Method for processing plant remains, in particular shells of seeds and nuts, even more in particular shells of cocoa beans, shells of grain seed and rice remains, residues of oil-containing germs and nuts, in particular according to Claims 1-7, with the following steps:
(i) generation of biogas in a biogas plant from at least some of the plant remains,
(ii) use of at least some of the biogas for providing energy during the production and/or processing of a product in which plant remains are left over, which for their part are processed at least partially in the method.

11. System for processing plant remains, in particular shells of seeds and nuts, even more in particular shells of cocoa beans, shells of grain seed and rice remains, remains of oil-containing germs and nuts, in particular according to Claim 10, comprising
- a production device, in particular a roaster or a roaster and a hydrolysis tank,
- a biogas tank,
- an input device, via which at least some of the plant remains can be transferred from the production device to the biogas tank and
- a biogas processing facility in which energy can be obtained from the biogas obtained in the biogas tank for operating the production facility.

## Revendications

1. Procédé pour le traitement de résidus végétaux, en particulier de coquilles de graines et de noix, plus particulièrement de coques de fèves de cacao, d'enveloppes de semences de céréales et de résidus de riz, de résidus de germes et de noix huileux, en particulier pour la production de fibres diététiques et/ou de substances de départ pour une installation de biogaz, **caractérisé par** les étapes de procédé suivantes
(i) mise à disposition de résidus végétaux présentant une proportion de coquilles d'au moins 20% en poids, en particulier d'au moins 30% en poids, plus particulièrement d'au moins 50% en poids, encore plus particulièrement d'au moins 90% en poids,
(ii) hydrolyse au moins partielle de constituants des résidus végétaux, en particulier hydrolyse au moins partielle d'un hydrate de carbone, d'une graisse et/ou d'une protéine,
(iii) séparation de la phase liquide présentant les constituants dissous et de la phase solide,
(iv) mise à disposition de la phase solide pour l'utilisation dans la production d'aliments, avantageusement par lavage, stérilisation et/ou séchage et en particulier par débactérisation.

2. Procédé selon la revendication 1, **caractérisé en ce qu'**on prépare, pour l'hydrolyse, une suspension des résidus végétaux dans un solvant, en particulier l'eau, présentant en particulier une proportion allant jusqu'à 40% en poids de masse sèche.

3. Procédé selon la revendication 1 ou 2, **caractérisé en ce que** l'hydrolyse a lieu dans une citerne à une température entre 25°C et 40°C, en particulier entre 30°C et 38°C.

4. Procédé selon l'une quelconque des revendications précédentes, **caractérisé en ce que** l'hydrolyse a lieu par voie enzymatique, en particulier avec addition d'enzymes.

5. Procédé selon l'une quelconque des revendications précédentes, **caractérisé en ce que** l'hydrolyse a lieu à un pH entre 3,0 et 6,5, de préférence entre 3,5 et 5,5, en particulier **en ce que** l'hydrolyse est réalisée avec addition d'un acide ou d'une base.

6. Procédé selon l'une quelconque des revendications précédentes, **caractérisé en ce qu'**on ajoute, pour l'hydrolyse, des micro-organismes, en particulier un inoculum.

7. Procédé selon l'une quelconque des revendications précédentes 1-6, **caractérisé en ce que** la phase liquide est transférée en tant que liquide riche en énergie dans une installation de biogaz.

8. Utilisation de la proportion solide du produit du procédé selon l'une quelconque des revendications 1-7 comme fibres diététiques.

9. Utilisation de la proportion liquide du produit du procédé pour le traitement de résidus végétaux, en particulier de coquilles de graines et de noix, plus particulièrement de coques de fèves de cacao, d'enveloppes de semences de céréales et de résidus de riz, de résidus de germes et de noix huileux, en particulier pour la production de fibres diététiques et/ou de substances de départ pour une installation de biogaz, présentant les étapes de procédé suivantes comme charge pour une installation de biogaz, en particulier pour la production de courant :
(i) mise à disposition de résidus végétaux présentant une proportion de coquilles d'au moins 20% en poids, en particulier d'au moins 30% en poids, plus particulièrement d'au moins 50% en poids, encore plus particulièrement d'au moins 90% en poids,
(ii) hydrolyse au moins partielle de constituants des résidus végétaux, en particulier hydrolyse au moins partielle d'un hydrate de carbone, d'une graisse et/ou d'une protéine.

10. Procédé pour le traitement de résidus végétaux, en particulier de coquilles de graines et de noix, plus particulièrement de coques de fèves de cacao, d'enveloppes de semences de céréales et de résidus de riz, de résidus de germes et de noix huileux, en particulier selon les revendications 1-7, présentant les étapes suivantes
(i) production d'un biogaz dans une installation de biogaz à partir d'au moins une partie des résidus végétaux,
(ii) utilisation d'au moins une partie du biogaz pour la mise à disposition d'énergie lors de la préparation et/ou du traitement d'un produit dans lequel il reste des résidus végétaux qui sont à leur tour traités au moins partiellement dans le procédé.

11. Installation pour le traitement de résidus végétaux, en particulier de coquilles de graines et de noix, plus particulièrement de coques de fèves de cacao, d'enveloppes de semences de céréales et de résidus de riz, de résidus de germes et de noix huileux, en particulier selon la revendication 10, comprenant
- un dispositif de production, en particulier un torréfacteur ou un torréfacteur et une citerne d'hydrolyse,
- une citerne de biogaz,
- un dispositif d'introduction via lequel au moins une partie des résidus végétaux provenant du dispositif de production peut être transférée dans la citerne de biogaz et
- un dispositif d'utilisation de biogaz permettant d'extraire de l'énergie pour exploiter le dispositif de production à partir du biogaz extrait dans la citerne de biogaz.
